(19) Europäisches Patentamt

European Patent Office

Office européen des brevets

(11) **EP 1 456 164 B1**

(12) **EUROPEAN PATENT SPECIFICATION**

(45) Date of publication and mention
of the grant of the patent:
**26.07.2006 Bulletin 2006/30**

(51) Int Cl.:
*C07C 51/487* (2006.01)    *C07C 63/00* (2006.01)
*C07C 63/16* (2006.01)    *C07C 63/26* (2006.01)
*C07C 63/24* (2006.01)    *C07C 63/38* (2006.01)

(21) Application number: 02805618.2

(22) Date of filing: **17.12.2002**

(86) International application number:
**PCT/US2002/040498**

(87) International publication number:
**WO 2003/053904 (03.07.2003 Gazette 2003/27)**

(54) **PURIFICATION OF A CRUDE ACID MIXTURE**

AUFREINIGUNG EINER ROHEN SÄUREMISCHUNG

PURIFICATION D'UN MELANGE D'ACIDES BRUTS

(84) Designated Contracting States:
**AT BE BG CH CY CZ DE DK EE ES FI FR GB GR
IE IT LI LU MC NL PT SE SI SK TR**

(30) Priority: **20.12.2001 US 342602 P
13.12.2002 US 318740**

(43) Date of publication of application:
**15.09.2004 Bulletin 2004/38**

(73) Proprietor: **BP Corporation North America Inc.
Warrenville, IL 60555 (US)**

(72) Inventor: **ROSEN, Bruce, I.
Park Ridge, IL 66068 (US)**

(74) Representative: **Hamer, Christopher K. et al
Mathys & Squire
120 Holborn
London EC1N 2SQ (GB)**

(56) References cited:
WO-A-00/00457        WO-A-99/29652
WO-A-02/088066        GB-A- 1 152 577
GB-A- 1 191 792

• **PATENT ABSTRACTS OF JAPAN vol. 2000, no.
05, 14 September 2000 (2000-09-14) -& JP 2000
037633 A (COSMO SOGO KENKYUSHO:KK), 8
February 2000 (2000-02-08)**
• **PATENT ABSTRACTS OF JAPAN vol. 1999, no.
03, 31 March 1999 (1999-03-31) -& JP 10 316613
A (HITACHI LTD), 2 December 1998 (1998-12-02)**

## Description

### Field of the Invention

The invention generally relates to a method for purifying a mixture of crude aromatic acids comprising at least one benzene carboxylic acid and at least one naphthalene carboxylic acid. The purification method of the present invention provides a purified aromatic acid mixture useful in the manufacture of ultraviolet light resistance polyesters.

### Background of the Invention

Benzene carboxylic acid is defined herein as a compound comprising a benzene ring substituted with at least two carboxylic acid groups. Examples of benzene carboxylic acids include phthalic acid, isophthalic acid, and terephthalic acid. Benzene carboxylic acids such as terephthalic acids are monomers and starting materials for various polyesters which are useful in a variety of applications including films, bottles, and other containers.

In certain applications, resistance to ultraviolet (UV) radiation (wavelengths within the range of 250 nm to 360 nm) is a desirable quality to impart to polyesters made from benzene carboxylic acids. UV resistance is especially desirable where these polyesters are used to preserve materials that are deleteriously affected by UV radiation such as foodstuffs, fruit juices, soft drinks, cosmetics, and pharmaceuticals. Because polyesters that incorporate naphthalene carboxylic acid monomers can provide superior UV resistance when compared to polyesters made from benzene carboxylic acids alone, making UV resistant polyester by using naphthalene dicarboxylic acid monomers along with benzene carboxylic monomers has been contemplated.

Naphthalene carboxylic acids are defined herein as compounds comprising a naphthalene ring substituted with at least two carboxylic groups. Examples of naphthalene carboxylic acids include naphthalene dicarboxylic acids (NDAs) such as 1,6 NDA, 2,7 NDA, 1,5 NDA, and 2,6 NDA.

A significant problem with using naphthalene carboxylic acids as monomers to impart UV resistance is the requirement of suitably pure monomer starting materials for the manufacture of polyesters. Crude benzene carboxylic acids are relatively easy to purify by dissolving in a solvent, hydrogenating, and then crystallizing. Crude naphthalene carboxylic acids are comparatively difficult to purify because of their low solubility in solvents. Purifying naphthalene carboxylic acids in the same manner as benzene carboxylic acids is thus not commercially feasible because of the costs associated with extremely high temperatures and pressures necessary to solubilize the naphthalene dicarboxylic acid. A solution to this problem is subjecting the crude naphthalene carboxylic acid (e.g. 2,6 naphthalene dicarboxylic acid) to an esterification process where it is reacted with an alcohol (e.g. methanol) to form the corresponding carboxylate (e.g. 2,6 naphthalene dicarboxylate) which may be purified by distillation. The purified carboxylate may then be subjected to hydrolysis to remove the alcohol and obtain a purified naphthalene dicarboxylic acid.

Because obtaining purified naphthalene dicarboxylic acid in this manner is both complicated and costly, hydrolysis is often not performed. Instead, the purified carboxylate (e.g. 2.6 naphthalene dicarboxylate) is used as a monomer starting material for polyester manufacture. For example, UK Patent Application (GB 2 222 940 A) discloses the use of 2,6 naphthalene dicarboxylate as a monomer to impart UV resistance. Unfortunately, during polyester manufacture, use of a carboxylate monomer results in the formation of an alcohol which is an undesirable waste product requiring further processing and/or disposal.

It would therefore be desirable to use a naphthalene carboxylic acid to impart the UV resistance (of its naphthalene ring) to a benzene carboxylic acid derived polyester while avoiding the costly purification of the naphthalene dicarboxylic acid, or esterification to make the corresponding naphthalene dicarboxylate.

### Brief Description of the Drawing

Figure 1 depicts the particle size distribution of purified terephthalic acid and a purified aromatic acid mixture in accordance with the present invention.
Figure 2 depicts the particle size distribution of purified terephthalic acid and a purified aromatic acid mixture in accordance with the present invention.
Figure 3 depicts the particle size distribution of purified terephthalic acid and two purified aromatic acid mixtures in accordance with the present invention.

### Summary of the Invention

Surprisingly, it has been discovered that the typical conditions for purification of benzene carboxylic acids may be used to co-purify a mixture of a crude benzene carboxylic acid and a crude naphthalene carboxylic. The method of the present invention involves the purification of a crude aromatic acid mixture comprising at least one crude benzene carboxylic acid and at least one crude naphthalene carboxylic acid, thereby producing a purified aromatic acid mixture. The purified aromatic acid mixture made by the method of the present invention may be used as monomer starting material for the manufacture of polymers with UV resistance imparted by incorporation of monomeric units with a naphthalene ring. The present invention may be employed to impart UV resistance while advantageously avoiding the separate and costly purification of a naphthalene dicarboxylic acid. The present invention may also be employed to impart UV resistance while advantageously avoiding the use of a naphthalene dicarboxylate which leads to the undesirable formation of an alcohol during polyester manufacture.

Crude aromatic acid mixture is defined herein as a mixture comprising at least one crude benzene carboxylic acid and at least one crude naphthalene carboxylic acid. Crude benzene carboxylic acid may be formed by oxidation of a benzene ring substituted with at least two oxidizable groups chosen from the group of alkyl, acyl, or combinations thereof. Crude naphthalene carboxylic acid may be provided by oxidation of a naphthalene ring substituted with at least two oxidizable groups chosen from the group of alkyl, acyl, or combinations thereof. Preferably, the crude benzene carboxylic acid and the crude naphthalene carboxylic acid are made by separate oxidation processes and are subsequently mixed to provide the crude aromatic acid mixture.

The method of purifying a crude aromatic acid mixture according to the present invention comprises feeding the crude aromatic acid mixture and a solvent thereof into a reactor having a hydrogenation catalyst. The reactor is maintained at a temperature and pressure so that the crude aromatic acid mixture remains solubilized. Hydrogen gas is fed into the reactor to produce a solubilized purified aromatic acid mixture. The pressure and temperature are reduced to crystallize the purified aromatic acid mixture which is subsequently recovered.

The crude benzene carboxylic acid may be chosen, for example, from phthalic acid, isophthalic acid, terephthalic acid, and mixtures thereof. The preferred crude benzene carboxylic acid is terephthalic acid. The crude naphthalene carboxylic acid may be chosen from 1,6 naphthalene dicarboxylic acid, 2,7 naphthalene dicarboxylic acid, 2,6 naphthalene dicarboxylic acid, isomers thereof, and mixtures thereof. The preferred crude naphthalene carboxylic acid is 2,6 naphthalene dicarboxylic acid.

The crude aromatic acid mixture should comprise at least 94 wt. % of a crude benzene carboxylic acid and no more than 6 wt. % of a crude naphthalene carboxylic acid. Preferably, the crude aromatic acid mixture should comprise at least 99 wt. % of a crude benzene carboxylic acid and no more than 1 wt. % of a crude naphthalene carboxylic acid. Most preferably, the crude aromatic acid mixture should comprise at least 99.5 wt. % of a crude benzene carboxylic acid and no more than 0.5 wt. % of a crude naphthalene carboxylic acid. To achieve a preferable UV resistance, the crude aromatic acid mixture should preferably comprise at least 0.25 wt. % of a crude naphthalene carboxylic acid.

A polyester with improved UV resistance may be made from the purified aromatic acid mixture of the present invention by reacting the purified aromatic acid mixture with a diol.

## Description of the Preferred Embodiment(s)

In accordance with the invention, a mixture of at least one crude benzene carboxylic acid and at least one crude naphthalene carboxylic acid (crude aromatic acid mixture) is co-purified by reduction of the impurities therein. The impurities present in the crude aromatic acid mixture may depend upon the oxidation reaction conditions used during their preparation, the aromatic feedstock selected, the oxidation catalyst or catalysts, and the levels of catalyst selected. The reaction mixture produced in the oxidation reaction contains, in addition to the desired aromatic carboxylic acid, a number of impurities and reaction by-products.

For example, when crude terephthalic acid is produced from oxidation of an aromatic feedstock comprising *para*-xylene, impurities of several types are present, including 4-carboxybenzaldehyde (4-CBA), an intermediate product in the oxidation of para-xylene. Unidentified color-forming precursors and color bodies, possibly of the fluorenone or anthraquinone structure, are also typically present. Typically, crude terephthalic acid has a 4-CBA content of about 3000 ppmw or higher. Acceptable levels of 4-CBA in starting materials for use in polyester manufacture are typically lower than 300 ppmw. Purity level may also be determined by measurement of L* and b* tri-stimulus color measurements as explained in detail below. Typically, the color values of crude terephthalic acid are L* less than 97 and b* greater than 2. Acceptable color values in starting materials for use in polyester manufacture typically are L* greater than 97 and b* less than 2.

When crude 2,6 naphthalene dicarboxylic acid is produced from an aromatic feedstock comprising 2,6-dimethylnaphthalene by use of a catalyst comprising cobalt, manganese and bromine components, impurities of several types are present. Such impurities typically include trimellitic acid (TMLA), bromo-2,6-naphthalenedicarboxylic acid (BrNDA), 2-formyl-6-naphthoic acid (2-FNA). and 2-naphthoic acid (2-NA). Typically, crude naphthalene dicarboxylic acid has a FNA content of about 50 ppmw or higher, and a BrNDA content of about 80 ppmw or higher. Typically, acceptable levels of these impurities in starting- materials for use in polyester manufacture are a FNA content lower than 5 ppmw, and a BrNDA content lower than 25 ppmw. Purity level may also be determined by measurement of L* and b* tri-stimulus color measurements as explained in detail below. Typically, the color values of crude naphthalene dicarboxylic acid are L* less than 97 and b* greater than 2. Acceptable color values in starting materials for use in polyester manufacture are L* greater than 97 and b* less than 2.

In accordance with the invention, the crude aromatic acid mixture comprising at least one crude benzene carboxylic acid and at least one crude naphthalene carboxylic acid is subjected to a co-purification process which is conducted at an elevated temperature and pressure in a pressure reaction vessel, in the presence of hydrogen gas and a catalyst comprising a solid carrier (preferably carbon) having one or more active catalytic hydrogenation components. The crude aromatic acid mixture is dissolved in a purification solvent (preferably water or a like polar solvent) to form a solution to yield desired weight percentages of crude benzene carboxylic acid and crude naphthalene carboxylic acid. The crude aromatic acid mixture is then hydrogenated and cooled to crystallize a purified crude aromatic acid mixture. In this manner, the invention is capable of reducing impurities and improving the color of the crude aromatic acid mixture to acceptable levels for use in typical polyester manufacturing process, i.e. 4-CBA content less than 300 ppmw, FNA content less than 5 ppmw, BrNDA content less than 25 ppmw, L* color value greater than 97 and b* color value less than 2.

Since benzene carboxylic acids are relatively soluble and naphthalene carboxylic acids are relatively insoluble, the crude aromatic acid mixture should comprise at least 94 wt. % of a crude benzene carboxylic acid and no more than 6 wt. % of a crude naphthalene carboxylic acid so that the resulting crude aromatic acid mixture has suitable solubility in the temperature range of 525-550 °F (274-287 °C). Use of more than 6 wt. % of a crude naphthalene carboxylic acid adversely affects the solubility of the resulting crude aromatic acid mixture in the temperature range of 525-550 °F (274-287 °C).

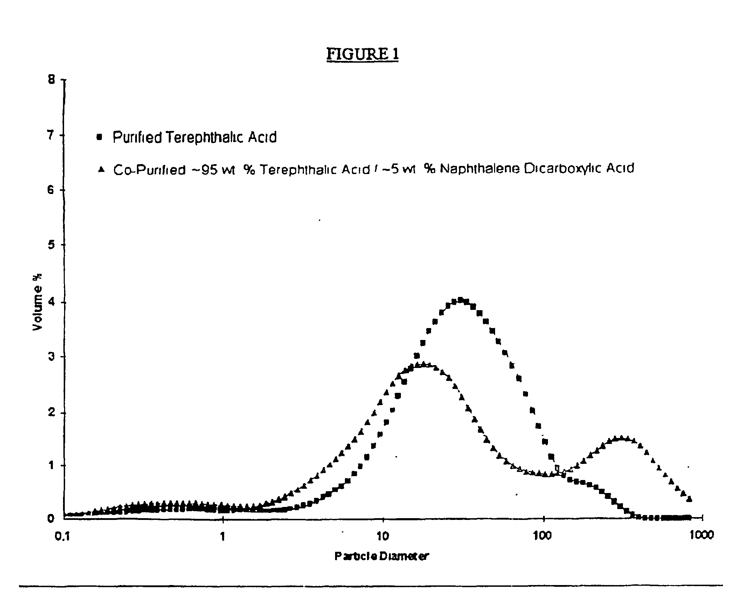

When a purified aromatic acid mixture has been made from purification of a crude aromatic acid mixture comprising about 94 wt. % of a crude benzene carboxylic acid (e.g. terephthalic acid) and about 6 wt % of a crude naphthalene carboxylic acid (e.g. 2,6 naphthalene dicarboxylic acid), the resulting purified aromatic acid mixture has a substantially bi-modal particle size distribution as measured by a Coulter counter (model LS130) and exemplified in Figure I. In contrast, the particle size distribution of a purified benzene dicarboxylic acid (e.g. terephthalic acid) obtained by purification of crude benzene dicarboxylic acid alone is substantially mono-modal as exemplified in Figure 1.

Because polyester manufacturing processes are generally tailored to the use of the substantially mono-modal particle size distribution of a purified benzene dicarboxylic acid, it would be desirable to have this substantially mono-modal particle size distribution property in the purified aromatic acid mixture of the present invention. It has been discovered that to achieve this substantially mono-modal particle size, the crude aromatic acid mixture should preferably comprise at least 99 wt. % of a crude benzene carboxylic acid and no more than 1 wt. % of a crude naphthalene carboxylic acid. Most preferably, the crude aromatic acid mixture should comprise at least 99.5 wt. % of a crude benzene carboxylic acid and no more than 0.5 wt. % of a crude naphthalene carboxylic acid.

Although water is the preferred solvent, other suitable polar solvents include the relatively lower molecular weight alkyl carboxylic acids containing from 2 to 6 carbon atoms, typically acetic acid, either alone or admixed with water.

Reactor temperature and pressure are chosen so that the crude aromatic acid mixture remains dissolved in solution. Typically, in order to dissolve practical amounts of the crude aromatic acid mixture in an appropriate solvent (typically water), the temperature must be substantially above the boiling point of the solvent at atmospheric pressure. A typical temperature range is from 525-550 °F (274-287 °C). It is typically necessary to employ process pressures considerably above atmospheric. If the reactor is hydraulically full, the reactor pressure may be controlled by the feed pumping rate. If the reactor has a headspace, the reactor pressure may be maintained by gaseous hydrogen alone or in admixture with an inert gas such as water vapor and/or nitrogen in the headspace. In general, the reactor pressure during hydrogenation may be in the range of 900 to 1500 pounds per square inch gauge (6205-10340 kPa), and usually is in the range of 900 to 1,300 pounds per square inch gauge (6205-8963 kPa).

In accordance with the invention, the solution of crude aromatic carboxylic acid is preferably treated with hydrogen in a pressure reactor vessel in a first reaction zone containing a hydrogenation catalyst. The hydrogenation catalyst typically comprises one or more active hydrogenation catalyst components supported on a carrier material. The carrier material is typically in a granular form although pellets or other types of particulate forms may be used. When in a granular form, the granules have an average size of -2 mesh to -12 mesh (U.S. Sieve Series), more preferably -4 mesh to -8 mesh. The carrier material is preferably an active carbon, and is more preferably derived from coconut charcoal. Such active carbon typically has a surface area of at least 600 $m^2$/gram ($N_2$; BET Method) preferably 800 $m^2$/gram to 1500 $m^2$/gram. While active carbon derived from coconut charcoal in the form of granules is preferred as a support material for the hydrogenation catalyst component, other porous carbonaceous supports or substrates may be used.

The hydrogenation catalyst used in the co-purification process of this invention contains at least one active catalytic hydrogenation component. Particularly suitable catalytic hydrogenation components are the Group VIII metals of the Periodic Table of Elements (IUPAC version), including palladium, platinum, rhodium, osmium, ruthenium, iridium, and mixtures thereof. The catalytic hydrogenation catalyst component may be deposited on, or added to the carbon carrier material by any standard method known in the art, for example, by treating the carbonaceous carrier with a solution of one or more soluble Group VIII metal compounds such as palladium chloride, and then drying the catalyst to remove excess solvent.

A suitable loading of the Group VIII metal on the carbon carrier is in the range of 0.01 weight percent to 2 weight percent based on the total weight of the finished catalyst, i.e. the total weight being the weight of the dry carbon carrier and the active hydrogenation component. Preferably, the Group VIII metal loading on the carbon carrier is 0.2 to 0.8 weight percent.

Suitable catalyst and catalyst beds useful in the invention for the purification of a crude aromatic acid mixture are described, for example, in U.S. Pat. Nos. 4,394,299; 4,629,715, 4,728,630 and 4,892,972. A suitable palladium-on-carbon catalyst may be obtained, for example, from Engelhard Corporation, Edison, N.J. under the designation "Palladium on Activated Carbon Granules (Carbon Code CG-21)." Also, suitable rhodium-on-carbon catalysts may be obtained from Engelhard Corporation, under the designation "Rhodium on Activated Carbon Granules (Carbon Code CG-21)."

The reactor used in the method of this invention is any reactor vessel that can withstand the temperature and pressure used for the hydrogenation of the crude aromatic acid mixture in solution. A preferred reactor configuration is a cylindrical reactor positioned vertically and having the hydrogenation catalyst contained therein in a fixed bed. In the preferred mode of operation, the crude aromatic acid mixture in solution is added to the reactor vessel at a position at or near the top portion of the reactor vessel and the crude aromatic acid mixture in solution flows down through the bed of hydrogenation catalyst contained in the reactor vessel in the presence of hydrogen gas wherein impurities in the solution are reacted with hydrogen gas. In this preferred mode, the crude aromatic acid mixture is purified and the purified product (co-purified acid mixture) is removed from the reactor vessel at a position at or near the bottom of the reactor.

In a suitable reactor vessel apparatus for the co-purification process of the invention, a hydrogenation catalyst comprising a carbon carrier and an active hydrogenation catalyst component is held within the reactor vessel by a screen or other means that retains the catalyst particles in the reactor, yet alloys the relatively free passage of the solution of the crude aromatic acid mixture in the purification solvent. The means used for retaining the catalyst particles may be a flat mesh screen or a screen made by closely spaced parallel wires. Other suitable catalyst retaining means include, for example, a tubular Johnson screen or a perforated plate. The means used for retaining the catalyst particles must be constructed of a material that is suitably resistant to corrosion and it must also be of an appropriate strength to efficiently retain the catalyst bed. Most suitably, the means used for retaining the catalyst bed has openings of 1 mm or less and is constructed of a metal such as stainless steel, titanium or Hastelloy C, commercially available from Haynes International, Inc of Kokomo, Indiana.

The reactor employed in the co-purification method of this invention may be operated in several modes. For example, a predetermined liquid level may be maintained in the reactor and hydrogen may be fed in. for any given reactor pressure, at a rate sufficient to maintain the predetermined liquid level. The difference between the actual reactor pressure and the vapor pressure of the crude aromatic acid mixture solution present is the hydrogen partial pressure in the reactor vapor space. Alternatively, if hydrogen is fed in admixture with an inert gas such as nitrogen, the difference between the actual reactor pressure and the vapor pressure of the crude acid solution present is the combined partial pressure of hydrogen and the inert gas admixed therewith. In this case the hydrogen partial pressure may be calculated from the known relative amounts of hydrogen and inert gas present in the admixture. In yet another operating mode, the reactor may, be filled with the solution of aromatic acid so as to provide no reactor vapor space. That is, the reactor may be operated as a hydraulically full system with dissolved hydrogen being fed to the reactor by flow control. In such an instance, the solution hydrogen concentration may be modulated by adjusting the hydrogen flow rate to the reactor. If desired, a pseudohydrogen partial pressure value may be calculated from the solution hydrogen concentration which, in turn, may be correlated with the hydrogen flow rate to the reactor.

In the operating mode where process control is effected by adjusting the hydrogen partial pressure, the hydrogen partial pressure in the reactor preferably is in the range of 10 pounds per square inch gauge to 200 pounds per square inch gauge (69-1379 kPa), or higher, depending upon the service pressure rating of the reactor, the degree of contamination of the aforementioned crude aromatic acid mixture, the activity and age of the particular catalyst employed, and like processing considerations. In the operating mode where process control is effected by adjusting directly the hydrogen concentration in the feed solution, the latter usually is less than saturated with respect to hydrogen and the reactor itself is hydraulically full. Thus, an adjustment of the hydrogen flow rate to the reactor will result in the desired control of hydrogen concentration in the solution. In general, the amount of hydrogen to be supplied to the purification reactor under reaction conditions is, of course, sufficient to effect the desired hydrogenation.

The space velocity reported as weight of the crude aromatic acid mixture solution per weight of catalyst per hour in the purification step is typically from 1 hour$^{-1}$ to 25 hours$^{-1}$, preferably from 2 hours$^{-1}$ to 15 hours$^{-1}$. The residence time of the solution in the catalyst bed varies, depending upon the space velocity.

After hydrogenation, the treated solution of crude aromatic acid mixture solution from the reactor is cooled to a crystallization temperature. The crystallization temperature is sufficiently low (e.g. 160° C. or below) for crystallization of the co-purified acid mixture to occur thereby producing crystals within a mother liquor. The crystallization temperature is sufficiently high so that impurities and their reduction products remain dissolved in the mother liquor. Thereafter, the mother liquor containing the dissolved impurities and their reduction products is separated from the crystallized co-purified acid mixture, whereby a purified aromatic acid mixture in accordance with the invention is recovered.

A polyester with improved UV resistance may be made from the purified aromatic acid mixture of the present invention by reacting the purified aromatic acid mixture with a diol. In general, a method for using the purified aromatic acid mixture of the present invention to make a polyester comprises forming a prepolymer by heating a mixture comprising an aromatic acid mixture and a diol; and further heating the prepolymer to make a polyester while liberating and removing water from the reaction. The prepolymer is heated until the desired molecular weight or degree of polymerization is achieved.

Crude benzene carboxylic acids suitable for use in the present invention may be made b, any known method in the art. Typically, crude benzene carboxylic acids are made by the liquid phase catalytic oxidation of an aromatic feedstock comprising benzene having at least two oxidizable substituents chosen from the group of alkyl, acyl, or combinations thereof. Suitable alkyl and/or acyl groups have from 1 to 6 carbon atoms. Examples of crude benzene carboxylic acids suitable for the invention are crude acids of phthalic, isophthalic, and terephthalic resulting from the oxidation of *ortho*-xylene, *meta*-xylene, and *para*-xylene respectively. A preferred crude benzene carboxylic acid is crude terephthalic acid formed by oxidation of *para*-xylene.

Crude naphthalene carboxylic acids suitable for use in the present invention may be made by any known method in the art. Typically, crude naphthalene carboxylic acids are made by the liquid phase catalytic oxidation of an aromatic feedstock comprising naphthalene having at least two oxidizable substituents chosen from the group of alkyl, acyl, or combinations thereof. Suitable alkyl and/or acyl groups have from 1 to 6 carbon atoms. Examples of crude naphthalene carboxylic acids suitable for the invention are crude acids obtained by the oxidation of 1,2-dimethyl naphthalene, 2,6-dialkyl naphthalene or 2-acyl-6-alkyl naphthalene, 2,6-dimethyl naphthalene, 2,6-diethyl naphthalene or 2,6-diisopropyl naphthalene, 2-acetyl-6-methyl naphthalene and 2-methyl-6-ethyl naphthalene. A preferred crude naphthalene carboxylic acid is 2,6-naphthalene dicarboxylic acid formed from the oxidation of 2,6-dialkyl naphthalene.

Typical solvents for use in the liquid phase catalytic oxidation to form the crude benzene carboxylic acids and/or crude naphthalene carboxylic acids suitable for the invention include water and any aliphatic $C_2$-$C_6$, monocarboxylic acid such as acetic acid, proprionic acid, n-butyric acid, isobutyric acid, n-valeric acid, trimethylacetic acid and caproic acid, and mixtures thereof. Preferably, the solvent is a mixture of acetic acid and water, which more preferably contains from 1 to 20 weight percent of water as introduced into an oxidation reactor. Since heat generated in the highly exothermic liquid-phase oxidation is dissipated at least partially by vaporization of solvent in the oxidation reactor, some of the solvent is withdrawn from the reactor as a vapor, which is typically then condensed and recycled to the reactor. In addition, some solvent is withdrawn from the reactor as a liquid in the product stream. After separation of the crude acid product from the product stream, at least a portion of the mother liquor (solvent) in the resulting product stream is typically recycled to the reactor.

Oxygen is used in the liquid phase catalytic oxidation to form the crude benzene carboxylic acids and/or crude naphthalene carboxylic acids suitable for the invention. The source of oxygen typically varies in molecular oxygen content from that of air to oxygen gas with air being the preferred source. In order to avoid the formation of explosive mixtures, the oxygen-containing gas fed to the reactor should provide an exhaust gas-vapor mixture containing from 0.5 to 8 volume percent oxygen (measured on a solvent-free basis). For example, a feed rate of the oxygen-containing gas sufficient to provide oxygen in the amount of from 1.5 to 2.8 moles per methyl substituent of a *meta* or *para*-xylene or dimethylnaphthalene being oxidized will provide such 0.5 to 8 volume percent of oxygen (measured on a solvent-free basis) in the gas-vapor mixture in the condenser.

The catalyst employed in the liquid phase catalytic oxidation to form the crude benzene carboxylic acids and/or crude naphthalene carboxylic acids suitable for the invention comprises a heavy metal component, and may additionally comprise promoters or accelerators known in the art. Suitable heavy metal oxidation catalysts typically include those metals having an atomic number of 21 to 82, inclusive. A promoter such as a suitable source of bromide, a low molecular weight ketone having from 2 to 6 carbon atoms or a low molecular weight aldehyde having 1 to 6 carbon atoms may also be used. The catalyst preferably comprises a cobalt containing component. More preferably, the catalyst further comprises a manganese containing component. Even more preferably, the catalyst further comprises a bromine containing component.

Typically, cobalt is present as a catalyst component so that the ratio of cobalt component (based on elemental cobalt) to feedstock is in the range of from 0.2 to 30 milligram atoms (mga) per gram mole of the aromatic feedstock. Typically, manganese is present as a catalyst component so that the ratio of manganese component (based on elemental manganese) to the cobalt component (based on elemental cobalt) is in the range of from 0.2 to 30 mga per mga of the cobalt component (based on elemental cobalt). Typically, bromine is present as a catalyst component so that the weight ratio of the bromine component (based on elemental bromine) to the cobalt component (based on elemental cobalt) and the manganese component (based on elemental manganese) is in the range of from 0.2 to 1.5 mga per mga of total cobalt component and manganese component (based on total elemental cobalt and elemental manganese).

Each of the cobalt and manganese components may be provided in any of their known ionic or combined forms that provide reactive forms of cobalt, manganese, and bromine in the solvent in the reactor. For example, when the solvent is an acetic acid medium, cobalt and/or manganese carbonate, acetate tetrahydrate, and/or bromide may be employed. Sources for bromine include elemental bromine ($Br_2$), or ionic bromide (for example, HBr, NaBr, KBr, $NH_4$, Br, etc.), or organic bromides which are known to provide bromide ions at the operating temperature of the oxidation (e.g., bromobenzenes, benzylbromide, mono- and dibromoacetic acid, bromoacetyl bromide, tetrabromoethane, ethylenedibromide, and the like). The bromine ion released from the organic bromides at the oxidation operating conditions may be readily determined by known analytical means. Tetrabromoethane, for example, at operating temperatures of 170° to 225° C. has been found to yield 3 effective gram atoms of bromine per gram mole.

In operation, the typical minimum pressure at which the oxidization reactor is maintained is that pressure required to keep the aromatic feedstock and at least 70 percent of the solvent substantially in the liquid phase. The aromatic feedstock and solvent not in the liquid phase because of vaporization is removed from the oxidation reactor as a vapor-gas mixture and typically is condensed and then returned to the oxidation reactor. When the solvent is an acetic acid-water mixture, suitable reaction gauge pressures in the oxidation reactor are in the range of from 0 kg/cm$^2$ to 35 kg/cm$^2$, and typically are in the range of from 10 kg/cm$^2$ to 30 kg/cm$^2$. The temperature range within the oxidation reactor is typically from 120° C., preferably from 150° C., to 240° C., preferably to 230° C. Typically, the solvent residence time in the oxidation reactor is from 20 to 150 minutes and preferably from 30 to 120 minutes.

The oxidation may be performed either in a batch, continuous, or semi continuous mode. In the batch mode, the aromatic feedstock to be oxidized, solvent, and the catalyst components are initially introduced batchwise into the reactor, and the temperature and pressure of the reactor contents are then raised to the desired levels for the commencement of the oxidation reaction. Air is introduced continuously into the reactor. After commencement of the oxidation reaction, for example, after all of the aromatic feedstock to be oxidized has been completely introduced into the reactor, the temperature of the reactor contents is raised. In the continuous mode, each of the aromatic feedstock to be oxidized, air, solvent, and catalyst are continuously introduced into the oxidation reactor, and a product stream comprising the resulting crude acid oxidation product and catalyst components dissolved in the solvent is withdrawn from the reactor. In the semi continuous mode, the solvent and catalyst are initially introduced into the reactor and subsequently, the aromatic feedstock to be oxidized and air are continuously introduced into the reactor.

For large-scale commercial operation, it is preferable to use a continuous oxidation process. Typically, in such a process, the weight ratio of monocarboxylic acid solvent to the aromatic feedstock to be oxidized is preferably 2:1 to 12:1. The mga ratio of manganese catalyst component to cobalt catalyst component (based on elemental cobalt and elemental manganese) is typically from 15:1 to 0.3:1. The mga ratio of bromine catalyst component to the total of cobalt catalyst component and manganese catalyst component is typically from 0.3:1 to 0.8:1 (calculated based on elemental bromine, elemental cobalt and elemental manganese). The total of cobalt catalyst component and manganese catalyst component (calculated based on elemental cobalt and elemental manganese) is typically at least 0.40 weight percent based on the weight of the solvent. The oxidation reaction temperature is typically 185° C to 250° C. Acetic acid is the most suitable solvent for such preferred continuous oxidation.

The crude aromatic acid mixture used in the co-purification process of this invention is preferably formed by mixing at least one crude benzene carboxylic and at least one crude naphthalene carboxylic acid which are in turn respectively formed by separate liquid phase catalytic oxidation processes. Alternatively. the crude benzene carboxylic and crude naphthalene carboxylic acid may be respectively made in the same liquid phase catalytic oxidation process, i.e. a co-oxidation process.

The invention will be more clearly understood from the following specific examples.

## Examples

Described below are specific non-limiting examples of the invention. The examples show the co-purification of a crude acid mixture comprising a crude benzene carboxylic acid and a crude naphthalene carboxylic acid. The result of co-purification in accordance with the invention yields a product with improved color properties and reduced levels of impurities such as 4-CBA, FNA, and BrNDA. The examples also show that a substantially mono-modal particle size distribution may be obtained by the co-purification method of the present invention.

## Color

The color was evaluated in all of the examples below by Tri-stimulus Color measurements. L, a* and b*. The measurement of the b*-value of a solid on the Hunter Color Scale is described in Hunter, The Measurement of Appearance, Chapter 8, pp. 102-132, John Wiley & Sons, N.Y., N.Y. (1975), and in Wyszecki et al., Color Science, Concepts and Methods. Quantitative Data and Formulae. 2d Ed. pp. 166-168, John Wiley & Sons, N.Y., N.Y. (1982).

More specifically, the b*-value of a sample was determined using a Diano Match Scan Spectrophotometer as follows. A sample was pressed into a pellet by placing 0.5 grams of the sample into a 13 mm mold and applying 4000 psi pressure for at least 90 seconds. The pellet was then irradiated with white light that was UV-filtered. The spectrum of the visible light reflected from the sample was determined and Tri-stimulus values (X, Y, and Z) were computed using the CIE Standard Observer functions. Using the weight-ordinate method. Tristimulus values are obtained from the following equations:

$$X = \sum_{400}^{700} R_1 x_\lambda$$

$$Y = \sum_{400}^{700} R_1 y_\lambda$$

$$Z = \sum_{400}^{700} R_1 z_\lambda$$

Where $R_1$ is the percent reflectance of the object at wavelength 1 and $x_\lambda$, $y_\lambda$ and $z_\lambda$ are the Standard Observer functions at wavelength 1 for CIE Illuminant D65. The Tri-stimulus values, X, Y, and Z, identify the color of the object in terms of the mixture of the primary lights that match it visually. Tri-stimulus values, however, are of limited use as color specifications because they do not correlate with visually meaningful attributes of color appearance and are not uniform in the spacing of colors as related to visual differences. As a result, "Uniform Color Scales" (UCS) have been adopted which use simple equations to approximate visual response. The UCS scale used by the Diano instrument is the CIE 1976 L*a*b* formula which converts Tri-stimulus values to L*, a*, and b* values as shown below:

$$L^* = 25[(100Y/Y_0)^{1/3} - 16$$

$$a^* = 500[(X/X_0)^{1/3} - (Y/Y_0)^{1/3}]$$

$$b^* = 200[(Y/Y_0)^{1/3} - (Z/Z_0)^{1/3}]$$

The L*-value is a measure of the luminosity or whiteness of an object where L*=100 is pure white, L*=0 is black. and in between is gray. The L*-value is strictly a function of the Tri-stimulus Y-value. The *a value measures the green and red of the green-ness to red-ness material attribute where positive a*-values represent green appearance and negative a*-values represent red appearance. The b*-value is a measure of the yellowness to blueness attribute where positive b*-values represent yellow appearance and negative b*-values represent blue appearance. The b*-value is a function of both Tri-stimulus values Y and Z.

## Crude Terephthalic Acid

The crude terephthalic acid (TA) used in all the of examples was obtained by the liquid phase catalytic oxidation of para-xylene. The crude TA had a 4-carboxybenzaldehyde (4-CBA) content of 4,690 ppmw as determined by liquid chromatography. The crude TA had color values of L* = 97.37, a* = -1.75, and b* = 6.02.

## Crude Naphthalene Dicarboxylic acid

The crude naphthalene dicarboxylic acid (NDA) used in all the examples was obtained by the liquid phase catalytic oxidation of dimethyl naphthalene. The crude NDA had a 2-formyl-6-naphthoic acid (FNA) content of 1,477 ppmw and a bromo-2,6 naphthalenedicarboxylic acid (BrNDA) content of 1,120 ppmw as determined by liquid chromatography.

## Crude Acid Purification Process

A continuous purification process was used in all of the examples and was performed by dissolving the crude acid or crude aromatic acid mixture in water to achieve a feed having 10 wt. % solids. The feed was then fed into a fixed reactor bed at a feed rate of 10 grams per minute. The reactor was operated at 1300 psig and 550 ° F. Hydrogen gas as fed into the reactor at the rate of 3-6 ml per minute. The catalyst used in the reactor bed was 0.5 wt. % Pd and 0.1 wt. % Sn supported on nutshell carbon commercially obtained from Engelhard of Iselin. New Jersey. This catalyst was washed with NaOH to remove impurities and the final Pd content to 0.4 wt. %. The reactor effluent was filtered at 160 ° F thereby recovering solids.

## Particle Size Distribution

In all of the examples, a Coulter counter (model LS 130) was used to determine particle size distributions. Coulter is a forward light scattering instrument designed to measure particle size distribution of a particular sample. About 30-50 grams of sample were measured for the examples below.

## Comparative Example 1

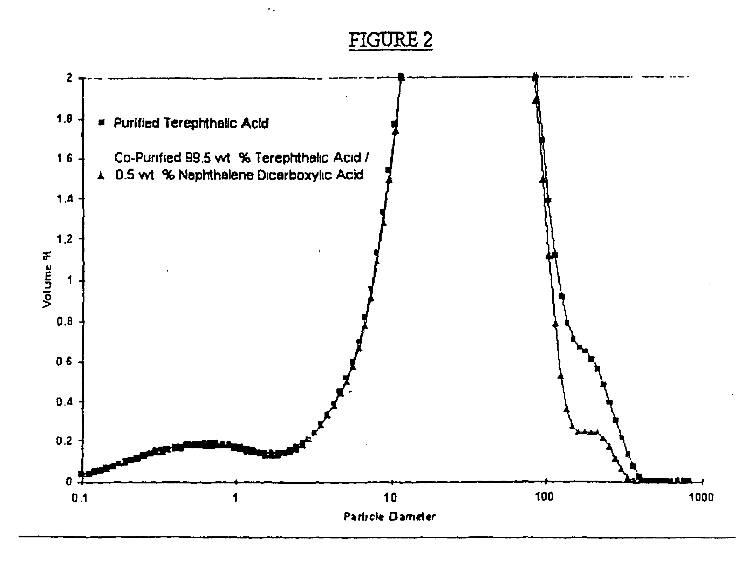

Crude TA was subjected to the purification process described above. Before purification is crude TA had a 4-carboxybenzaldehyde (4-CBA) content of 4,690 ppmw as determined by liquid chromatography and color values of L*=97.37, a*=-1 .75. b*=6.02. After purification, the resulting purified TA had a reduced 4-carboxybenzaldehyde (4-CBA) content of 349 ppmw as determined by liquid chromatography and improved color values of L*=97.37, a*=-0.82, b*=3.47. The particle size distribution of the purified TA is depicted in Figures 1-3. As seen by Figures 1-3, the particle size distribution of the purified TA is substantially mono-modal.

## Example 2

Crude PTA and crude NDA was physically blended in amounts to yield a crude aromatic acid mixture comprising: 94.22 wt. % TA, 5.36 wt. % NDA. 3,980 ppmw 4-CBA, 69 ppmw FNA and 85 ppmw BrNDA as determined by liquid chromatography. The color values of the crude aromatic acid mixture were L*=92.11, a*=0.90, b*=9.66. This crude aromatic acid mixture was subjected to the purification process described above and the resulting purified acid mixture had reduced impurities of: 243 ppmw 4-CBA, 3 ppmw FNA and 19 ppmw BrNDA. The color values of the resulting purified acid mixture were improved to L*=98.2, a*=-0.49, b*=2.13. The particle size distribution of the purified acid mixture is depicted in Figure 1. As shown by Figure 1, the particle size distribution of a purified acid mixture comprising about 95 wt. % TA and 5 wt. % NDA is substantially bi-modal when compared to the particle size distribution of purified TA alone.

## Example 3

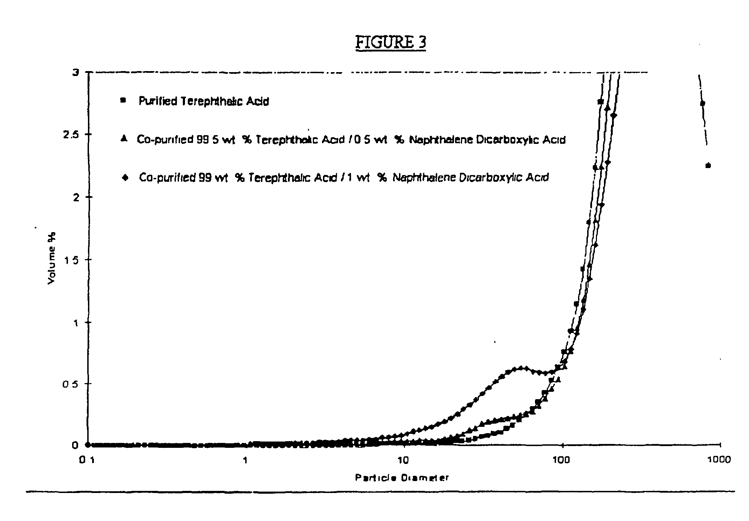

Crude PTA and crude NDA was physically blended in amounts to yield a crude aromatic acid mixture comprising about 99.5 wt. % TA and about 0.5 wt. % NDA. This crude aromatic acid mixture was subjected to the purification process described above. The particle size distribution of the purified acid mixture is depicted in Figure 3. As shown by Figure 3, the particle size distribution of a purified acid mixture comprising about 99.5 wt. % TA and 0.5 wt. % NDA is substantially mono-modal and similar to the particle size distribution of purified TA alone.

## Example 4

Crude PTA and crude NDA was physically blended in amounts to yield a crude aromatic acid mixture comprising about 99 wt. % TA and about 1 wt.% NDA. This crude aromatic acid mixture was subjected to the purification process described above. The particle size distribution of the purified acid mixture is depicted in Figure 3. As shown by Figure 3, the particle size distribution of a purified acid mixture comprising about 99 wt. % TA and 1 wt. % NDA is substantially mono-modal and similar to the particle size distribution of purified TA alone.

## Claims

1. A method of purifying a mixture of at least one crude benzene carboxylic acid and at least one crude naphthalene carboxylic acid, said method comprising hydrogenating the mixture in the presence of hydrogen and a hydrogenation catalyst to produce a purified aromatic acid mixture.

2. The method of claim 1 wherein the crude aromatic acid mixture, a solvent thereof, and hydrogen are fed into to a reactor containing a hydrogenation catalyst.

3. The method of claim 1 wherein the crude aromatic acid mixture comprises:

   (i) at least 94 weight percent benzene carboxylic acid comprising a benzene ring substituted with at least two carboxylic acid groups; and
   (ii) no more than 6 weight percent a naphthalene carboxylic acid comprising a naphthalene ring substituted with at least two carboxylic acid groups.

4. The method of claim 2 wherein the reactor is maintained at a temperature and pressure wherein the crude aromatic acid mixture is solubilized by the solvent.

5. The method of claim 4 wherein the purified aromatic acid is crystallized by cooling to a crystallization temperature.

6. The method of claim 5 wherein the purified aromatic acid is separated from the solvent.

7. The method of claim 3 wherein the benzene carboxylic acid is chosen from phthalic acid, isophthalic acid, terephthalic acid, and mixtures thereof.

8. The method of claim 3 wherein the naphthalene carboxylic acid is chosen from 1,6 naphthalene dicarboxylic- acid, 2,7 naphthalene dicarboxylic acid, 2,6 naphthalene dicarboxylic acid, isomers thereof, and mixtures thereof.

9. The method of daim 1 wherein the crude aromatic acid mixture comprises terephthalic acid and 2,6 naphthalene dicarboxylic acid.

10. The method of claim 1 wherein the crude aromatic acid mixture comprises no more that 0.5 wt. % naphthalene carboxylic acid.

11. The method of claim 4 wherein the temperature is maintained at 525-550 °F (274-287 °C) and the pressure is maintained at 900-1300 pounds per square inch gage (6205-8963 kPa).

**12.** The method of claim 1 wherein the crude aromatic acid mixture comprises at least 99 weight percent benzene carboxylic acid and no more than 1 weight percent naphthalene carboxylic acid.

**13.** The method of claim 1 wherein the crude aromatic acid mixture comprises at least 99.5 weight percent benzene carboxylic acid and no more than 0.5 weight percent naphthalene carboxylic acid.

**14.** The method of claim 2 wherein the solvent is chosen from the group of water, alkyl carboxylic acids containing from 2 to 6 carbon atoms, and mixtures thereof.

**15.** The method of claim 1 wherein the hydrogenation catalyst comprises a catalytic hydrogenation component supported by a carrier material wherein the catalytic hydrogenation component is chosen from the group of palladium, platinum, rhodium, osmium, ruthenium, iridium, and mixtures thereof.

**16.** The method of claim 1 wherein the crude aromatic acid mixture comprises greater than 3000 ppmw 4-CBA, greater than 50 ppmw FNA, and greater than 80 ppmw BrNDA.

**17.** The method of claim 1 wherein the crude aromatic acid mixture has a tristimulus L\* color value less than 97 and a b\* color value greater than 2.

**18.** The method of claim 1 wherein the purified aromatic acid mixture comprises less than 300 ppmw 4-CBA, less than 5 ppmw FNA, and less than 25 ppmw BrNDA.

**19.** The method of claim 1 wherein the purified aromatic acid mixture has a tristimulus L\* color value greater than 97 and a b\* color value less than 2.

**20.** A method of polmerization comprising forming a prepolymer by heating a mixture comprising a diol and a purified aromatic acid mixture obtained by the method of Claim 1; and further heating the prepolymer to make a polyester while liberating and removing water from the reaction.

**Patentansprüche**

**1.** Verfahren zur Reinigung einer Mischung aus wenigstens einer hohlen Benzolcarbonsäure und wenigstens einer rohen Naphtalincarbonsäure, wobei das Verfahren die Hydrierung der Mischung in Anwesenheit von Wasserstoff und einem Hydrierungskatalysator zur Erzeugung einer gereinigten aromatischen Säuremischung umfasst.

**2.** Verfahren nach Anspruch 1, wobei die rohe aromatische Säuremischung, ein Lösungsmittel dafür und Wasserstoff in einen Reaktor, der einen Hydrierungskatalysator enthält, gegeben werden.

**3.** Verfahren nach Anspruch 1, wobei die rohe aromatische Säuremischung enthält:

(I) wenigstens 94 Gewichtsprozent Benzolcarbonsäure, die einen mit wenigstens zwei Carbonsäuregruppen subsituierten Benzolring enthält; und
(II) nicht mehr als 6 Gewichtsprozent einer Naphtalincarbonsäure, die einen mit wenigstens zwei Carbonsäuregruppen substituierten Naphtalinring enthält.

**4.** Verfahren nach Anspruch 2, wobei der Reaktor auf einer Temperatur und einem Druck gehalten wird, bei denen die rohe aromatische Säuremischung von dem Lösungsmittel in Lösung gebracht wird.

**5.** Verfahren nach Anspruch 4, wobei die gereinigte aromatische Säure durch Abkühlen auf eine Kristallisationstemperatur auskristalliert wird.

**6.** Verfahren nach Anspruch 5, wobei die gereinigte aromatische Säure von dem Lösungsmittel getrennt wird.

**7.** Verfahren nach Anspruch 3, wobei die Benzolcarbonsäure aus Phthalsäure, Isophthalsäure, Terephthalsäure und Mischungen hieraus ausgewählt ist.

**8.** Verfahren nach Anspruch 3, wobei die Naphtalincarbonsäure aus 1,6-Naphtalindicarbonsäure, 2,7-Naphtalindicarbonsäure, 2,6-Naphtalindicarbonsäure, Isomeren hieraus und Mischungen hiervon ausgewählt ist.

**9.** Verfahren nach Anspruch 1, wobei die rohe aromatische Säuremischung Terephthalsäure und 2,6-Naphtalindicarbonsäure enthält.

**10.** Verfahren nach Anspruch 1, wobei die rohe aromatische Säuremischung nicht mehr als 0,5 Gew.-% Naphtalincarbonsäure enthält.

**11.** Verfahren nach Anspruch 4, wobei die Temperatur bei 525-550 °F (274-287 °C) unter Druck bei 900-1300 per square inch gage (6205-8963 kPa) gehalten wird.

**12.** Verfahren nach Anspruch 1, wobei die rohe aromatische Säuremischung wenigstens 99 Gewichtsprozent Benzolcarbonsäure und nicht mehr als 1 Gewichtsprozent Naphtalincarbonsäure enthält.

13. Verfahren nach Anspruch 1, wobei die rohe aromatische Säuremischung wenigstens 99,5 Gewichtsprozent Benzolcarbonsäure und nicht mehr als 0,5 Gewichtsprozent Naphtalincarbonsäure enthält.

14. Verfahren nach Anspruch 2, wobei das Lösungsmittel aus der Gruppe: Wasser, Alkylcarbonsäuren mit 2 bis 6 Kohlenstoffatomen, und Mischungen hieraus ausgewählt ist.

15. Verfahren nach Anspruch 1, wobei der Hydrierungskatalysator eine katalytische Hydrierkomponente enthält, die von einem Trägermaterial gestützt ist, wobei die katalytische Hydrierkomponente aus der Gruppe: Palladium, Platin, Rhodium, Osmium, Ruthenium, Iridium und Mischungen hieraus ausgewählt ist.

16. Verfahren nach Anspruch 1, wobei die rohe aromatische Säuremischung mehr als 3000 ppmw 4-CBA, mehr als 50 ppmw FNA, und mehr als 80 ppmw BrNDA enthält.

17. Verfahren nach Anspruch 1, wobei die rohe aromatische Säuremischung einen Tristimulus L* Farbwert von weniger als 97 und einen a b*-Farbwert größer als 2 hat.

18. Verfahren nach Anspruch 1, wobei die gereinigte aromatische Säuremischung weniger als 300 ppmw 4-CBA, weniger als 5 ppmw FNA und weniger als 25 ppmw BrNDA enthält.

19. Verfahren nach Anspruch 1, wobei die gereinigte aromatische Säuremischung einen Tristimulus L* Farbwert größer als 97 und einen a b*-Farbwert von weniger als 2 hat.

20. Polymerisationsverfahren, enthaltend die folgenden Schritte:

Man bildet ein Vorpolymer durch Erhitzen einer Mischung, die ein Diol und eine gereinigte aromatische Säuremischung, welche nach dem Verfahren des Anspruchs 1 erhalten wurde, enthält; und man erhitzt weiterhin das Vorpolymer durch Herstellung eines Polyesters, während man Wasser aus der Reaktion freisetzt und entfernt.

**Revendications**

1. Procédé de purification d'un mélange d'au moins un acide benzoïque brut et d'au moins un acide naphtoïque brut, ledit procédé comprenant l'étape consistant à hydrogéner le mélange en présence d'hydrogène et d'un catalyseur d'hydrogénation pour produire un mélange d'acide aromatique purifié.

2. Procédé selon la revendication 1, dans lequel le mélange d'acide aromatique brut, un solvant de celui-ci, et de l'hydrogène sont introduits dans un réacteur contenant un catalyseur d'hydrogénation.

3. Procédé selon la revendication 1, dans lequel le mélange d'acide aromatique brut comprend :

(i) au moins 94 % en poids d'acide benzoïque comprenant un cycle benzénique substitué par au moins deux groupes acide carboxylique ; et (ii) pas plus de 6 % en poids d'un acide naphtoïque comprenant un cycle naphtalène substitué par au moins deux groupes acide carboxylique.

4. Procédé selon la revendication 2, dans lequel le réacteur est maintenu à une température et une pression dans lesquelles le mélange d'acide aromatique brut est solubilisé par le solvant.

5. Procédé selon la revendication 4, dans lequel l'acide aromatique purifié est cristallisé en refroidissant à une température de cristallisation.

6. Procédé selon la revendication 5, dans lequel l'acide aromatique purifié est séparé du solvant.

7. Procédé selon la revendication 3, dans lequel l'acide benzoïque est choisi parmi l'acide phtalique, l'acide isophtalique, l'acide téréphtalique, et les mélanges de ceux-ci.

8. Procédé selon la revendication 3, dans lequel l'acide naphtoïque est choisi parmi l'acide 1,6-naphthalène dicarboxylique, l'acide 2,7-naphthalène dicarboxylique, l'acide 2,6-naphthalène dicarboxylique, leurs isomères, et les mélanges de ceux-ci.

9. Procédé selon la revendication 1, dans lequel le mélange d'acide aromatique brut comprend de l'acide téréphtalique et de l'acide 2,6-naphtalène dicarboxylique.

10. Procédé selon la revendication 1, dans lequel le mélange d'acide aromatique brut ne comprend pas plus de 0,5 % en poids d'acide naphtoïque.

11. Procédé selon la revendication 4, dans lequel la température est maintenue à 274-287° C et la pression manométrique est maintenue à 6 205-8 963 kPa.

12. Procédé selon la revendication 1, dans lequel le mélange d'acide aromatique brut comprend au moins 99 % en poids d'acide benzoïque et pas plus de 1 % en poids d'acide naphtoïque.

13. Procédé selon la revendication 1, dans lequel le mélange d'acide aromatique brut comprend au moins 99,5 % en poids d'acide benzoïque et pas plus de 0,5 % en poids d'acide naphtoïque.

14. Procédé selon la revendication 2, dans lequel le solvant est choisi dans le groupe de l'eau, des acides carboxyliques alkyliques contenant de 2 à 6 atomes de carbone, et des mélanges de ceux-ci.

15. Procédé selon la revendication 1, dans lequel le catalyseur d'hydrogénation comprend un composant d'hydrogénation catalytique supporté par un matériau support dans lequel le composant d'hydrogénation catalytique est choisi dans le groupe du palladium, du platine, du rhodium, de l'osmium, du ruthénium, de l'iridium, et des mélanges de ceux-ci.

16. Procédé selon la revendication 1, dans lequel le mélange d'acide aromatique brut comprend plus de 3 000 ppm en poids de 4-CBA, plus de 50 ppm en poids de FNA, et plus de 80 ppm en poids de BrNDA.

17. Procédé selon la revendication 1, dans lequel le mélange d'acide aromatique brut a une valeur de la composante trichromatique L* inférieure à 97 et une valeur chromatique b* supérieure à 2.

18. Procédé selon la revendication 1, dans lequel le mélange d'acide aromatique purifié comprend moins de 300 ppm en poids de 4-CBA, moins de 5 ppm en poids de FNA, et moins de 25 ppm en poids de BrNDA.

19. Procédé selon la revendication 1, dans lequel le mélange d'acide aromatique purifié a une valeur de la composante trichromatique L* supérieure à 97 et une valeur chromatique b* inférieure à 2.

20. Procédé de polymérisation comprenant l'étape consistant à former un prépolymère en chauffant un mélange comprenant un diol et un mélange d'acide aromatique purifié obtenu par le procédé de la revendication 1 ; et en chauffant davantage le prépolymère pour faire un polyester tout en libérant et en éliminant l'eau de la réaction.

FIGURE 1

FIGURE 1

■ Purified Terephthalic Acid

▲ Co-Purified ~95 wt % Terephthalic Acid / ~5 wt % Naphthalene Dicarboxylic Acid

EP 1 456 164 B1

## FIGURE 2

EP 1 456 164 B1

## FIGURE 3